# EUROPEAN PATENT SPECIFICATION

(11) **EP 1 882 936 B1**
(45) Date of publication and mention of the grant of the patent: **14.09.2011**
(21) Application number: 06015674.2
(22) Date of filing: 27.07.2006
(51) Int. Cl.: G01N 33/50, G01N 33/543

(54) **Urinary immunochromatographic detection cup**
Gefäß für immunochromatographischen Nachweis im Harn
Ventouse de détection immuno-chromatographique urinaire

(43) Date of publication of application: 30.01.2008
(73) Proprietor: AraGen Biotechnology Co. Ltd., 11710 Naor (JO)
(72) Inventor: Adnan, Badwan, Dr., 11185 Amman (JO); Murshed, Abedel-qader Mohammed, 11118 Amman (JO)
(74) Representative: Winkler, Andreas Fritz Ernst

(56) References cited:
- DE-A1- 2 826 651
- US-A- 4 624 929
- US-B1- 6 203 757
- PATENT ABSTRACTS OF JAPAN vol. 1996, no. 11, 29 November 1996 (1996-11-29) & JP 08 193994 A (SEKISUI CHEM CO LTD), 30 July 1996 (1996-07-30)
- PATENT ABSTRACTS OF JAPAN vol. 1995, no. 03, 28 April 1995 (1995-04-28) & JP 06 331625 A (SEKISUI CHEM CO LTD), 2 December 1994 (1994-12-02)

## Description

### Background of the Invention

Testing for HIV is an essential component in the diagnosis and treatment of persons infected with the virus, in screening of blood for transfusion, in surveillance and in HIV/AIDS related research. Thus, accurate and cost-effective testing is of great importance in combating the spread of HIV. It is imperative that tests for the diagnosis of HIV infection be as accurate as possible, given the serious ethical, legal and social issues that accompany HIV infection¹.

The use of body fluids other than blood as specimens for detecting antibodies to HIV has been reported to have potential as an alternative medium for HIV testing. The use of urine samples may be attractive due to the ease of sample collection, possible cost savings, better safety (against needle stick injuries) and higher compliance rates. Assays for these types of specimens can be a useful alternative when it is difficult or impossible to test for HIV in blood samples. It may be that blood cannot be drawn for religious reasons or difficulties may be experienced in collecting blood samples in hard to reach places where it is, nevertheless, important to have epidemiological surveillance¹. Moreover, urinary samples are the more safety type of specimens in case of HIV infection.

Today new HIV tests have been developed for use with urine specimens. Urine contains very low levels of IgG, frequently around 1 mg/l, and therefore very sensitive techniques are required to detect specific antibody. Thus, to date, the tests for urine specimens have been based on ELISA and Western blot techniques, however, efforts are being made to develop simple and/or rapid tests for the detection of antibody to HIV in urine specimens ¹. However, it would be of particular advance to have rapid immunochromatographic test devices available for detecting HIV antibodies in urine.

In recent years the *in vitro* diagnostics (IVD) industry has made enormous efforts to develop immunochromatographic tests. Such tests have found applications in both clinical and non-clinical fields ². A clinical utility of this test format has been shown for more than 150 different analytes, and many of them are target now of commercially available diagnostic products ³. The wide range of applications for such devices has been reviewed ^{2,4}.

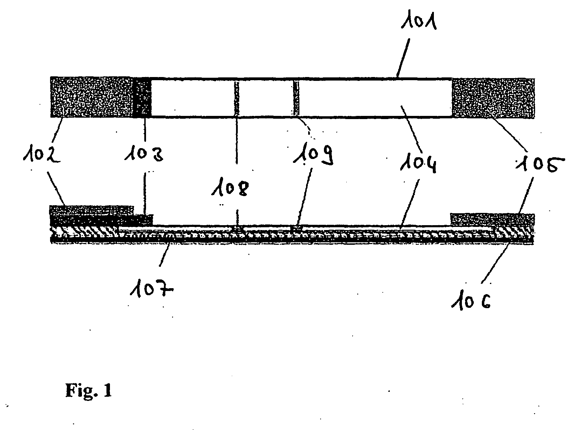

Rapid immunochromatographic test devices, e.g. in the form of a test strip, are made up of a number of components (see Fig. 1). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102.The sample then flows through the sample pad 102 into the conjugate pad 103, where it mixes with and releases the detector reagent. This mixture then flows across the membrane 104, where it binds with the test and control reagents located in the capture test zone 108 and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

The object of the present invention is to provide a rapid immunochromatographic test device for detecting antibodies such as HIV antibodies in urine specimens.

US 6 203 757 describes the use of an anti-IgG collodial gold conjugate in immunochromatographic devices using urine as a sample.

### Summary of the Invention

The object of the present invention is solved by a urinary immunochromatographic detection cup comprising:
(a) a sample-collecting container;
(b) at least one test device inside the container, which test device comprises at least one capture antigen recognized by an antibody to be detected and/or at least one control antibody;
(c) at least one conjugate releasing pad separated from the test device, which conjugate releasing pad comprises at least one anti-IgG colloidal gold conjugate and optionally modified water-soluble chitosan;
(d) at least two sample absorbent pads linked to the test device at different positions.

In one embodiment, the test device comprises a nitrocellulose membrane on which the capture antigen and/or the control antibody are immobilized.

In one embodiment, the antibody to be detected is a human antibody selected from HIV IgG, HCV IgG, and *H. pylori* IgG.

In one embodiment, the capture antigen is selected from envelop protein gp 160 from HIV, NS3 antigen from HCV, envelop antigen(s) from HCV, and *H. pylori* antigen(s).

In one embodiment, the control antibody is anti-mouse IgG.

In one embodiment, the anti-IgG colloidal gold conjugate is mouse anti-human IgG colloidal gold conjugate.

In one embodiment, the conjugate releasing pad is located at the internal surface of the container wall.

In one embodiment, a first sample absorbent pad is located at the bottom of the container and a second sample absorbent pad is located at the top of the container.

In a preferred embodiment, the absorbent sample pad at the top is fitted into a container cap.

In a particularly preferred embodiment, the absorbent sample pad at the top has the capacity of absorbing at least 15 ml of sample.

The object of the present invention is further solved by a use of the urinary immunochromatographic detection cup for detecting a human antibody selected from HIV IgG, HCV IgG, and *H. pylori* IgG in a sample of urine of a subject, preferably human.

In one embodiment, the volume of the sample of urine sample is approximately 15 ml.

In one embodiment, the detection cup is used for simultaneously detecting HIV IgG and/or HCV IgG.

In another embodiment, the detection cup is used simultaneously detecting of HIV IgG, and/or HCV IgG, and *H. pylori* IgG.

In one embodiment, the detection cup is used for the diagnosis and monitoring of a subject infected with HIV, HCV and/or *H. pylori.*

The object of the present invention is further solved by a method for preparing the urinary immunochromatographic detection cup according to the present invention, wherein the mixture of at least one anti-IgG colloidal gold conjugate and optionally modified water-soluble chitosan is prepared by contacting the chitosan and a colloidal gold prior to conjugation of the colloid gold with an anti-IgG.

In one embodiment, the colloidal gold is prepared by reduction of 1% aqueous solution of tetrachloroauric acid using trisodium citrate aqueous solution to produce spheroidal gold particles.

Thus, the present invention provides a rapid immunochromatographic test device for detecting HIV, HCV and/or *H. pylori* antibodies in a patient's sample readily obtainable by non-invasive procedures, namely urine. The detection of IgG in urine requires a high sensitivity which is achieved by essentially two mechanisms.

First, the use of a large volume of urine as a sample (about 15 ml) instead of 17 µl of serum (concentration of IgG in urine is about 1:6000) reduced the factor to 1:7.

Second, the colloidal gold conjugate colour intensity was amplified using water-soluble chitosan (or modified water-soluble chitosan) as a colour intensity modification agent. The action of water-soluble chitosan (or modified water-soluble chitosan) is on the colour intensity of the colloidal gold. It is added during the preparation of colloidal gold, but before the conjugation of colloidal gold with anti-human IgG. Water-soluble chitosan (or modified water-soluble chitosan) affect the colour intensity of colloidal gold and so increase the ability of the human eye to identify the colour, and, thus, enable to detect very low concentrations. Signal amplification lies in the range of up to 10folds. Colloidal gold could be prepared by the reduction of 1% aqueous solution of tetrachloroauric acid (HAuCl₄) using trisodium citrate aqueous solution to produce spheroidal gold particles. After colloidal gold preparation, water-soluble chitosan (or modified water-soluble chitosan) aqueous solution was added with a suitable volume and concentration to convert colour from purple to violet depending on the volume and concentration of the added modification solution.

### Detailed Description of the Invention

### Brief Description of the Figures

Figure 1 shows top and side views of a typical rapid-flow immunochromatographic test device in the form of a test strip 101 including a sample pad 102, a conjugate pad 103, a membrane 104, an absorbent pad 105, an adhesive 106, a supporting backing 107, a test zone 108, and a control zone 109.
Figure 2a shows an assembly comprising a test strip 201, an absorbent sample pad 202, and an absorbent pad 205.
Figure 2b shows an immunochromatographic detection cup comprising a sample-collecting container 210, a cap 211 and the assembly shown in Fig. 2a.

### EXAMPLES

### EXAMPLE 1: Urinary Immunochromatographic Detection Cup

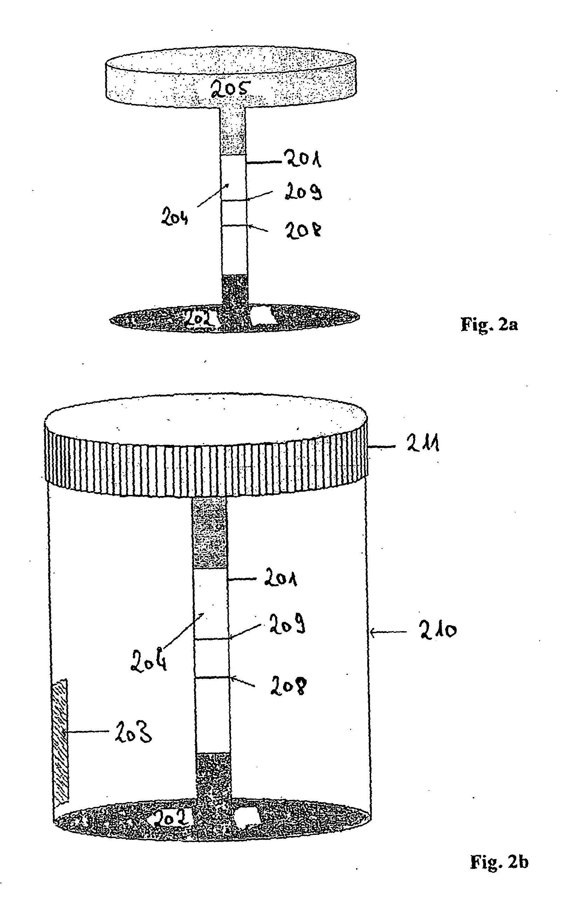

One embodiment of the rapid immunochromatographic detection system of the present invention is shown in Fig. 2a, b. The immunochromatographic detection cup comprises a sample-collecting container 210, actually the "cup", a cap 211 for closing the container, and a detection test strip 201 inserted into the container 210. The test strip 201 may be placed inside the transparent container wall, and the test result can be read on the outer surface. The test strip 201 comprises a nitrocellulose membrane 204 and is linked via an absorbent pad 202 on the test strip 201 to an absorbent sample pad 202 placed on the bottom of the container 210. The absorbent sample pad 202 is designed to cover the inner surface of the container bottom. At the opposite end, the test strip 201 is linked via an absorbent pad 205 on the test strip to an absorbent pad 205 which is fitted into the cap 211 of the container and is designed to be thick enough to absorb more than 15 ml of sample. An anti-human immunoglobulin gold conjugate releasing pad 203 is fixed to the internal surface of the container wall. The anti-human immunoglobulin gold conjugate will begin releasing during the urine sample streaming into the container 210. Non-specific antibody is immobilized as a control zone 209 onto the nitrocellulose membrane 204 for non-specific capturing of the anti-human immunoglobulin gold conjugate, while antigens are immobilized as a sample zone 208 for specific capturing of the antibody to be detected. The antigens may be synthetic and/or recombinant. In the embodiment shown in Fig. 2a, b the sample zone 208 and the control zone 209 are realized by a sample and control line, respectively. More than one sample zone 208 and/or more than one control zone 209 are also contemplated.

### EXAMPLE 2: Urinary HIV IgG Detection Cup

Mouse anti-human IgG colloidal gold conjugate is comprised by the conjugate releasing pad 203 immobilized onto the internal surface of the sample-collecting container 210. The sample zone 208 is comprises recombinant envelop protein gp160 immobilized onto the nitrocellulose membrane 204. The control zone 209 comprises anti-mouse IgG. Sample zone 208 and control zone 209 turn into purple colour in case that HIV IgG is present in the sample; only the control zone 209 turns into purple colour in case of HIV IgG free sample.

### EXAMPLE 3: Urinary HCV IgG Detection Cup

Mouse anti-human IgG colloidal gold conjugate is comprised by the conjugate releasing pad 203 immobilized onto the internal surface of the sample-collecting container 210. The sample zone 208 comprises a mixture of synthetic and recombinant HCV NS3 and HCV envelop antigens_immobilized onto the nitrocellulose membrane 204. The control zone 209 comprises anti-mouse IgG. Sample zone 208 and control zone 209 turn into purple colour in case that HCV IgG is present in the sample; only the control zone 209 turns into purple colour in case of HCV IgG free sample.

### EXAMPLE 4: Urinary H. pylori IgG Detection Cup

Mouse anti-human IgG colloidal gold conjugate is comprised by the conjugate releasing pad 203 immobilized onto the internal surface of the sample-collecting container 210. The sample zone 208 comprises a mixture of synthetic and recombinant *H. pylori* antigens immobilized onto the nitrocellulose membrane 204. The control zone 209 comprises anti-mouse IgG. Sample zone 208 and control zone 209 turn into purple colour in case that *H. pylori* IgG is present in the sample; only the control zone 209 turns into purple colour in case of *H. pylori* IgG free sample.

### EXAMPLE 5: Urinary HIV/HCV IgG Detection Cup

Mouse anti-human IgG colloidal gold conjugate is comprised by the conjugate releasing pad 203 immobilized onto the internal surface of the sample-collecting container 210. A first sample zone 208 comprises recombinant HIV envelop protein gp160 immobilized onto the nitrocellulose membrane 204. A second sample zone 208' comprises a mixture of synthetic and recombinant HCV NS3 and HCV envelop antigens immobilized onto the nitrocellulose membrane 202. The control zone 209 comprises anti-mouse IgG. The first sample zone 308 and control zone 309 turn into purple colour in case that HIV IgG is present in the sample; the second sample zone 308' and control zone 309 turn into purple colour in case that HCV IgG is present in the sample; only the control zone 309 turns into purple colour in case of HIV IgG and HCV IgG free sample.

### EXAMPLE 6: Urinary HIV/HCV/H. pylori IgG Detection Cup

Mouse anti-human IgG colloidal gold conjugate is comprised by the conjugate releasing pad 203 immobilized onto the internal surface of the sample-collecting container 210. A first sample zone 308 is recombinant HIV envelop protein gp160 immobilized onto the nitrocellulose membrane 204. A second sample zone 208' comprises a mixture of synthetic and recombinant HCV NS3 and HCV envelop antigens immobilized onto the nitrocellulose membrane 204. A third sample zone 208" comprises synthetic and recombinant *H. pylori* antigens_immobilized onto the nitrocellulose membrane 204 The control zone 209 comprises anti-mouse IgG. The first sample zone 208 and the control zone 309 turn into purple colour in case that HIV IgG is present in the sample; the second sample zone 308'and the control zone turn into purple colour in case that HCV IgG is present in the sample; the third sample zone 208" and the control zone 209 turn into purple colour in case that *H. pylori* IgG is present in the sample; only the control zone 209 turns into purple colour in case of HIV IgG/HCV IgG/and *H*. *pylori* IgG free sample.

### References

(1) World Health Organization, HIV assays: operational characteristics (Phase I). Report 13: urine specimens, oral fluid (saliva) specimens. [Material originally distributed as WHO/BCT/02.08]
(2) J Chandler, N Robinson, and K Whiting, "Handling False Signals in Gold-Based Rapid Tests", IVD Technology 7, no. 2 (2001): 34-45; http://www.devicelink.com/ivdt/archive/01 /03/002.html.
(3) TC Tisone et al., "Image Analysis for Rapid-Flow Diagnostics", IVD Technology 5, no. 5 (1999): 52-58; http://www.devicelink.com/ivdtlarchive/99/09/010.html.
(4) J Chandler, T Gurmin, and N Robinson, "The Place of Gold in Rapid Tests", IVD Technology 6, no. 2 (2000): 37-49; http://www.devicelink.com/ivdt/archive/00/03/004.html

## Claims

1. An urinary immunochromatographic detection cup comprising:
(a) a sample-collecting container;
(b) at least one test device inside the container, which test device comprises at least one capture antigen recognized by an antibody to be detected and/or at least one control antibody;
(c) at least one conjugate releasing pad separated from the test device, which conjugate releasing pad comprises a mixture of at least one anti-IgG colloidal gold conjugate and water-soluble chitosan optionally modified
(d) at least two sample absorbent pads linked to the test device at different positions.

2. The detection cup according to claim 1, wherein the test device further comprises a nitrocellulose membrane on which the capture antigen and/or the control antibody is immobilized.

3. The detection cup according to claim 1 or 2, wherein the antibody to be detected is a human antibody selected from HIV IgG, HCV IgG, and *H*. *pylori* IgG.

4. The detection cup according to any of the preceding claims, wherein the capture antigen is selected from envelop protein gp 160 from HIV, NS3 antigen from HCV, envelop antigen from HCV, and *H. Pylori* antigen,

5. The detection cup according to any of the preceding claims, wherein the control antibody is anti-mouse IgG.

6. The detection cup according to any of the preceding claims, wherein the anti-IgG. colloidal gold conjugate is mouse anti-human IgG colloidal gold conjugate.

7. The detection cup according to any of the preceding claims, wherein the conjugate releasing pad is located at the internal surface of the container wall.

8. The detection cup according to' any of the preceding claims, wherein a first sample absorbent pad is located at the bottom and a second sample pad is located at the top of the container.

9. The detection cup according to claim 8, wherein the sample absorbent pad at the top is fitted into a container cap.

10. The detection cup according to claim 8 or 9, wherein the sample absorbent pad at the top has a capacity of absorbing at least 15 ml of sample.

11. A use of the urinary fluid immunochromatographic detection cup according to any of claims 1 to 10 for detecting a human antibody selected from HIV IgG, HCV IgG, and *H. pylori* IgG in sample of urine of a subject, preferably human.

12. The use according to claim 11, wherein the volume of the urine sample is approximately 15 ml.

13. The use according to claim 11 or 12 for simultaneously detecting HIV IgG and/or and HCV IgG.

14. The use according to claim 11 or 12 for simultaneously detecting HIV IgG and/or HCV IgG, and *H. pylori* IgG.

15. The use according to any of claims 11 to 14 for the diagnosis and monitoring of a subject infected with HIV, HCV and/or *H. pylori.*

16. A method for preparing the urinary fluid immunochromatographic detection cup according to any of claims 1 to 10, wherein the mixture of at least one anti-IgG colloidal gold conjugate and water-soluble chitosan optionally modified is prepared by contacting the chitosan and a colloidal gold prior to conjugation of the colloid gold with an anti-IgG.

17. The method according to claim 16, wherein the colloidal gold is prepared by reduction of 1% aqueous solution of tetrachloroauric acid using trisodium citrate aqueous solution to produce spheroidal gold particles.

## Patentansprüche

1. Gefäß für immunchromatographischen Nachweis im Harn, umfassend:
(a) einen Probensammelbehälter;
(b) wenigstens eine Testvorrichtung in dem Behälter, wobei die Testvorrichtung wenigstens ein Fänger-Antigen, welches von einem nachzuweisenden Antikörper erkannt wird, und/oder wenigstens einen Kontrollantikörper umfasst;
(c) wenigstens ein Konjugat-freisetzendes Kissen, getrennt von der Testvorrichtung, wobei das Konjugat-freisetzende Kissen eine Mischung von wenigstens einem Anti-IgG-kolloidalen Gold-Konjugat und wasserlösliches Chitosan, welches fakultativ modifiziert ist, umfasst;
(d) wenigstens zwei Proben-absorbierende Kissen, die mit der Testvorrichtung an verschiedenen Positionen verbunden sind.

2. Gefäß zum Nachweis nach Anspruch 1, wobei die Testvorrichtung weiterhin eine Nitrozellulose-Membran umfasst, auf der das Fänger-Antigen und/oder der KontrollAntikörper immobilisiert ist.

3. Gefäß zum Nachweis nach Anspruch 1 oder 2, wobei der nachzuweisende Antikörper ein humaner Antikörper ist, ausgewählt aus HIV-IgG, HCV-IgG und H. pylori-IgG.

4. Gefäß zum Nachweis nach einem der vorangehenden Ansprüche, wobei das Fänger-Antigen ausgewählt ist aus dem Hüllprotein gp 160 von HIV, NS3-Antigen von HCV, Hüllantigen von HCV und H. pylori-Antigen.

5. Gefäß zum Nachweis nach einem der vorangehenden Ansprüche, wobei der KontrollAntikörper Anti-Maus-IgG ist.

6. Gefäß zum Nachweis nach einem der vorangehenden Ansprüche, wobei das Anti-IgGkolloidale Gold-Konjugat ein Maus-anti-humanes IgG-kolloidales Gold-Konjugat ist.

7. Gefäß zum Nachweis nach einem der vorangehenden Ansprüche, wobei sich das Konjugat-freisetzende Kissen an der inneren Oberfläche der Behälterwand befindet.

8. Gefäß zum Nachweis nach einem der vorangehenden Ansprüche, wobei sich ein erstes Proben-absorbierendes Kissen am Boden befindet und ein zweites Probenkissen sich am oberen Teil des Behälters befindet.

9. Gefäß zum Nachweis nach Anspruch 8, wobei das Proben-absorbierende Kissen am oberen Teil in eine Behälterkappe eingepasst ist.

10. Gefäß zum Nachweis nach Anspruch 8 oder 9, wobei das Proben-absorbierende Kissen am oberen Teil eine Absorptionskapazität von wenigstens 15 ml Probe hat.

11. Verwendung eines Gefäßes zum immunochromatographischen Nachweis in Harnflüssigkeit nach einem der Ansprüche 1-10 zum Nachweis eines humanen Antikörpers, ausgewählt aus HIV-IgG, HCV-IgG und H. pylori-IgG in einer Urinprobe eines Patienten, bevorzugt eines humanen Patienten.

12. Verwendung nach Anspruch 11, wobei das Volumen der Urinprobe näherungsweise 15 ml ist.

13. Verwendung nach Anspruch 11 oder 12 zum simultanen Nachweis von HIV-IgG und/oder HCV-IgG.

14. Verwendung nach Anspruch 11 oder 12 zum simultanen Nachweis von HIV-IgG und/oder HCV-IgG und H. pylori-IgG.

15. Verwendung nach einem der Ansprüche 11 bis 14 zur Diagnose und Überwachung eines Patienten, der mit HIV, HCV und/oder H. pylori infiziert ist.

16. Verfahren zum Herstellen des Gefäßes zum immunochromatographischen Nachweis in Harnflüssigkeit nach einem der Ansprüche 1 bis 10, wobei die Mischung von wenigstens einem Anti-IgG-kolloidalen Gold-Konjugat und einem fakultativ modifizierten wasserlöslichen Chitosan hergestellt wird durch In-Kontakt-Bringen des Chitosans und einem kolloidalen Gold vor der Konjugation des kolloidalen Golds mit einem Anti-IgG.

17. Verfahren nach Anspruch 16, wobei das kolloidale Gold hergestellt wird durch Reduktion einer 1%igen wässrigen Lösung von Tetrachlorgoldsäure unter Verwendung von wässriger Trinatriumcitratlösung, um sphäroidale Goldpartikel zu erzeugen.

## Revendications

1. Coupe de détection urinaire immunochromatographique comprenant :
(a) un récipient de collecte d'échantillons ;
(b) au moins un dispositif de test à l'intérieur du récipient, lequel dispositif de test comprend au moins un antigène de capture reconnu par un anticorps à détecter et/ou au moins un anticorps de contrôle ;
(c) au moins un tampon de libération de conjugué séparé du dispositif de test, lequel tampon de libération de conjugué comprend un mélange d'au moins un conjugué d'or colloïdal anti-IgG et de la chitosane soluble dans l'eau, éventuellement modifiée ;
(d) au moins deux tampons d'absorption d'échantillons liés au dispositif de test à diverses positions.

2. Coupe de détection selon la revendication 1, dans laquelle le dispositif de test comprend en outre une membrane de nitrocellulose sur laquelle l'antigène de capture et/ou l'anticorps de contrôle est immobilisé.

3. Coupe de détection selon la revendication 1 ou 2, dans laquelle l'anticorps à détecter est un anticorps humain sélectionné parmi l'IgG anti-VIH, l'IgG anti-VHC, et l'IgG anti-H. pylori.

4. Coupe de détection selon l'une quelconque des revendications précédentes, dans laquelle l'antigène de capture est sélectionné parmi la protéine d'enveloppe gp 160 du VIH, l'antigène NS3 du VHC, l'antigène d'enveloppe du VHC, et l'antigène d'H. pylori.

5. Coupe de détection selon l'une quelconque des revendications précédentes, dans laquelle l'anticorps de contrôle est l'anticorps IgG anti-souris.

6. Coupe de détection selon l'une quelconque des revendications précédentes, dans laquelle le conjugué d'or colloïdal anti-IgG est un conjugué d'or colloïdal souris anti-IgG humaine.

7. Coupe de détection selon l'une quelconque des revendications précédentes, dans laquelle le tampon de libération de conjugué est situé au niveau de la surface intérieure de la paroi du récipient.

8. Coupe de détection selon l'une quelconque des revendications précédentes, dans laquelle un premier tampon d'absorption d'échantillons est situé au fond et un deuxième tampon d'échantillons est situé en haut du récipient.

9. Coupe de détection selon la revendication 8, dans laquelle le tampon d'absorption d'échantillons situé en haut est ajusté dans un couvercle du récipient.

10. Coupe de détection selon la revendication 8 ou 9, dans laquelle le tampon d'absorption d'échantillons situé en haut a une capacité d'absorption d'au moins 15 ml d'échantillon.

11. Utilisation de la coupe de détection immunochromatographique d'un fluide urinaire selon l'une quelconque des revendications 1 à 10 pour la détection d'un anticorps humain sélectionné parmi l'IgG anti-VIH, l'IgG anti-VHC et l'IgG anti-H. pylori dans un échantillon d'urine d'un sujet, de préférence, d'un sujet humain.

12. Utilisation selon la revendication 11, dans laquelle le volume de l'échantillon d'urine est d'approximativement 15 ml.

13. Utilisation selon la revendication 11 ou 12 pour détecter simultanément l'IgG anti-VIH et/ou l'IgG anti-VHC.

14. Utilisation selon la revendication 11 ou 12 pour détecter simultanément l'IgG anti-VIH et/ou l'IgG anti-VHC, et l'IgG anti-H. pylori.

15. Utilisation selon l'une quelconque des revendications 11 à 14 pour le diagnostic et la surveillance d'un sujet infecté par le VIH, le VHC et/ou H. pylori.

16. Procédé de préparation de la coupe de détection immunochromatographique d'un fluide urinaire selon l'une quelconque des revendications 1 à 10, dans lequel le mélange d'au moins un conjugué d'or colloïdal anti-IgG et de chitosane soluble dans l'eau, éventuellement modifiée, est préparé en mettant en contact la chitosane avec un conjugué d'or colloïdal avant la conjugaison de l'or colloïdal avec l'anti-IgG.

17. Procédé selon la revendication 16, dans lequel l'or colloïdal est préparé par la réduction d'une solution aqueuse d'acide tétrachloroaurique à 1% en utilisant une solution aqueuse de citrate trisodique pour produire des particules d'or sphéroïdales.
